# EUROPEAN PATENT SPECIFICATION

(11) **EP 1 437 597 B1**
(45) Date of publication and mention of the grant of the patent: **09.09.2015**
(21) Application number: 04000349.3
(22) Date of filing: 09.01.2004
(51) Int. Cl.: G01N 33/68, B01L 3/00

(54) **Protein measurement method in protein production plant by cell culture and apparatus thereof**
Methode und Vorrichtung zur Proteinbestimmung bei deren Herstellung in Zellkultur-Produktionsanlagen.
Méthode et dispositif pour la détermination de protéines lors de leur production dans des installations de production utilisant des cultures de cellules.

(30) Priority: 09.01.2003 JP 2003003322
(43) Date of publication of application: 14.07.2004
(73) Proprietor: Hitachi, Ltd., Chiyoda-ku Tokyo (JP)
(72) Inventor: Kondo, Takeyuki, Chiyoda-ku, Tokyo 100-8280 (JP); Nanba, Masaru, Chiyoda-ku, Tokyo 100-8280 (JP); Haga, Ryoichi, Chiyoda-ku, Tokyo 100-8280 (JP)
(74) Representative: Beetz & Partner mbB

(56) References cited:
- WO-A-00/70353
- US-A- 6 046 056
- US-A- 6 082 185
- GOTTSCHLICH N ET AL: "Integrated microchip-device for the digestion, separation and postcolumn labeling of proteins and peptides" JOURNAL OF CHROMATOGRAPHY. BIOMEDICAL APPLICATIONS, ELSEVIER, AMSTERDAM, NL, vol. 745, no. 1, 4 August 2000 (2000-08-04), pages 243-249, XP004215303 ISSN: 0378-4347
- PATENT ABSTRACTS OF JAPAN vol. 2002, no. 11, 6 November 2002 (2002-11-06) & JP 2002 207031 A (SHIMADZU CORP), 26 July 2002 (2002-07-26)

## Description

### BACKGROUND OF THE INVENTION

### (1) Field of the Invention

The present invention relates to a protein quantitative measurement method in a cell culture plant or the like, and an apparatus thereof.

### (2) Description of the Related Art

A polymer protein pharmaceutical such as an antibody is produced by culturing biological cells in a culture tank. Then, a pharmaceutical as a product is separated from a culture solution, refined and dispatched. Since the quality and safety of the pharmaceutical should be assured, it is required to check whether the pharmaceutical has been actually treated normally not only in the biological cell culture step but also in subsequent separation and refinement steps. In recent years, in particular, needs for quality assurance have grown since GMP (Good Manufacturing Practice for Pharmaceuticals) came into effect.

Hitherto, quantitative measurement of the polymer pharmaceutical has been performed in such a manner that an operator manually collects a culture solution or sample liquid from each operation step, and makes measurements by the ELISA (Enzyme-linked Immunosorbant Assay) method using micro-well plates. This assay operation has a large number of operation processes as described in "Immunology Illustrated" 81-W1362-02CU-SF/Kf (translated under supervision by Tomio Tada, issued by Nankodo, January 1, 2000).

Furthermore, since quantitative measurements should be made manually for a large number of sample liquids collected at fixed time intervals from a plurality of production steps subsequent to a culture step, the burden on a measurement operator is enormous. Furthermore, since samples are collected from a culture tank manually, external bacteria and viruses may enter the culture tank to infect or destruct culture cells.

Furthermore, in this method, an antigen-antibody reaction that is diffusion-controlled is made to proceed in a relatively large reaction field having a depth of about several millimeters, the measurement time required for obtaining an analysis result after taking a sample is therefore 3 hours or more, and thus the monitoring of culture states and the quality control of the product are not adequately performed. For performing the monitoring of culture states and high quality product control, time required for measurement should be reduced to about 20 minutes or less.

Methods enabling fast measurements and reducing the burden on operators may include an automatic measurement method using high pressure liquid chromatography described in "High pressure Liquid Chromatography Handbook", second edition revised (edited by Japan Society for Analytical Chemistry, Kanto Division, issued by Maruzen).

In this method, separation/quantitative measurement is carried out using adsorption/desorption of a protein to/from a column, but a high pressure pump is required because a sample liquid should be fed to fine adsorption columns. Furthermore, pressure-resistant parts should be used in tubing, thus bringing about the problem that an apparatus is prone to upsizing.

JP-A-2001-4628 discloses an immunoassay microchip in which a micro-channel reaction area and a micro-channel inflow area are formed on a glass substrate. The micro-channel reaction area is filled with solid particles, the antigen-antibody reaction is made to proceed on the solid particles, and an analysis is made according to photo-thermal conversion analysis.

Methods enabling fast measurements include a measurement method using a surface plasmon resonance (SPR) sensor as described in JP-A-2002-148258. In this method, an adsorption amount is measured using the shift of the resonance angle of a surface plasmon due to adsorption of a material on the surface of a metal thin film.

However, the resonance angle has very strong temperature dependency, thus there arises the problem that strict temperature control within ±0.1°C using a Peltier element or the like is required, and two sensors must be provided to make a reference measurement if temperature control cannot be performed. Furthermore, because of susceptibility to impurities, a sensor should be calibrated for each measurement, and errors are significant although sensitivity is high. Furthermore, a rotation device of a laser source is required for scanning the resonance angle, and thus the apparatus configuration is complicated and expensive.

Problems associated with SPR, conventional ELISA method and HPLC (high pressure liquid chromatography) described above are listed as follows.
(1) SPR: strict temperature control and calibration for each measurement are required, errors are significant, and the apparatus is complicated and expensive.
(2) Conventional ELISA method: the analysis speed is low.
(3) HPLC: upsizing is caused, and the analysis speed is low.

Document WO 00/70353 describes a method and apparatus for the quantitative measurement of proteins according to the preamble of claims 1 and 7.

### SUMMARY OF THE INVENTION

The object of the present invention is to overcome the drawbacks of the prior art and to provide a quantitative protein measurement apparatus and a measurement method enabling fast measurements and being free from contamination of each process device during collection of a sample liquid.

The above problem is solved according to the independent claims. The dependent claims relate to preferred embodiments of the concept of the present invention.

The method of the present invention for the quantitative measurement of proteins in a liquid sample is defined in claim 1.

The method of the present invention for the quantitative measurement of a protein in a liquid sample comprises the following steps:
- sampling a sample liquid online one or more times from a flow including
   - a cell culture step, wherein proteins, such as antibodies, enzymes or other proteins, are produced and secreted into the culture solution or are flowed out from dead or disrupted cells into the culture solution,
   - a separation step subsequent to the cell culture step and
   - a culture product purification step,
- performing a dilution and/or a filtration of the sample liquid to adjust the liquid composition,
- passing the sample liquid obtained by the adjustment through a microflow cell (microchip),
- allowing the protein contained in the sample liquid to undergo a coloring reaction with an enzyme or a reaction resulting in a product slowing fluorescence or phosphorescence, in the microflow cell, and
- measuring the amount of protein.

The apparatus of the present invention for the quantitative measurement of proteins in liquid samples, preferably for carrying out the above-defined methods, is defined in claim 7.

According to a preferred embodiment, the apparatus further comprises a recording unit for recording quantitative measurement results.

In accordance with another preferred embodiment, the control unit is designed to control all of the operations of the apparatus.

Other objects, features and advantages of the invention will become apparent from the following description of the embodiments of the invention taken in conjunction with the accompanying drawings.

### BRIEF DESCRIPTION OF THE DRAWINGS

Fig. 1 is a perspective view showing an outline of a microflow cell (microchip) in an automatic protein measurement apparatus according to the present invention.
Fig. 2 is a block diagram of one example of a system of the automatic protein measurement apparatus according to the present invention.
Fig. 3 is a block diagram illustrating a specific configuration of a measurement unit that is used in the system of the present invention.
Fig. 4 is a block diagram illustrating means for switching between LDH measurement and antibody measurement that is used in the system of the present invention.
Fig. 5 is a block diagram illustrating the quantitative protein measurement method by fluorometry or phosphorometry that is used in the system of the present invention.
Fig. 6 is a graph showing comparison of analysis results from the method of the present invention and the conventional ELISA method.

### PREFERRED EMBODIMENT OF THE INVENTION

An automatic protein measurement apparatus according to the present invention comprises a sampling unit for taking a sample liquid online at least one time from a flow including a cell culture step (wherein an antibody and other proteins are produced and secreted into the culture medium), a separation step subsequent to said cell culture and a culture product purification step in a plant for producing biological products such as protein, a preprocessing unit for performing at least one of dilution and filtration of the sample liquid to adjust the liquid composition so that the sample liquid can be passed through a microflow cell (microchip), a microflow cell (microchip) for making a protein contained in the adjusted liquid undergo a reaction with an enzyme, a measurement unit for quantitatively measuring the protein according to a result obtained from the reaction, a control unit for automatically controlling a series of operations of the above units constituting the apparatus, and a recording unit for recording a quantitative measurement result.

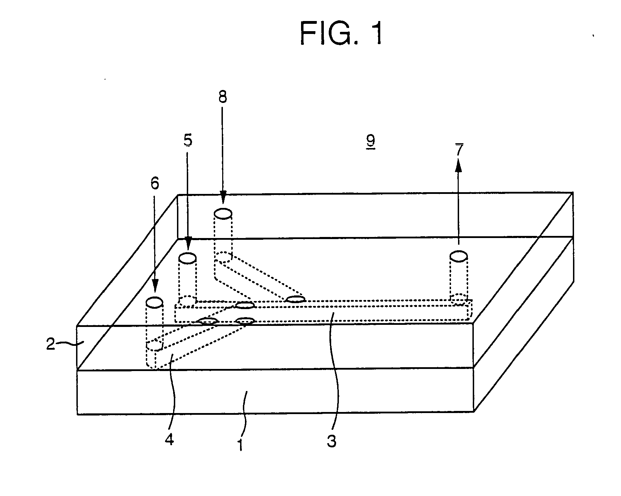

In the present invention, a micro-channel reaction apparatus described in JP-A-2001-4628 can be used as a microflow cell (microchip) for making a protein undergo a reaction with an enzyme to develop a color. Furthermore, the microflow cell (microchip) shown in Fig. 1 can be used.

Use of the microflow cell (microchip) can reduce reaction time considerably compared with use of liquid chromatography. The cross-sectional area of the reaction area of the microflow cell (microchip) is 0.04 mm² or less. If the cross-sectional area is larger, the reaction time is prolonged and the reliability of an online monitor in a culture plant or the like is compromised.

As shown schematically in Fig. 1, the microflow cell (microchip) for use in the present invention is comprised of upper and lower two substrates 1 and 2. Micro-channels 3, 4 are formed on the lower substrate, and a sample and a reagent are fed through tubes 5, 6. The sample that has undergone a predetermined reaction is fed through tubes 7, 8 to an optical detection unit.

This microflow cell (microchip) is disposable, so that it is discarded after each reaction, and replaced with a new flow cell (microchip) when the sample is changed. The cross-sectional area of the micro-channel is 0.04 mm² or less in relation to the reaction speed.

Thus, the sample must be passed through a very narrow flow path reaction unit, and it is therefore impossible to feed the reaction sample in its original form. Thus, it is required to dilute or filter the collected sample in advance so that the sample can pass through the narrow reaction path. In the present invention, both dilution and filtration may be performed as a matter of course.

In an analysis method described in JP-A-2001-4628, a photo-thermal conversion analysis using a gold colloid as a labelled antibody and using a thermal lens microscope is used, but because a light source for exciting the colloid is used, there arises the problem that the apparatus is complicated and expensive. In the present invention, an enzyme and a protein are made to undergo a coloring reaction, and they are detected by an optical system, thus making it possible to extremely simplify the apparatus configuration and reduce the cost.

In the present invention, a protein-enzyme of interest and its substrate solution are made to contact each other while making them to flow at almost a constant rate in the microflow cell (microchip), whereby they are converted into a coloring pigment. Then, a quantitative measurement is made with the absorbance at a specified wavelength or the fluorescence color level. This process is used for detection of LDH (lactate dehydrogenase) and the like.

A primary antibody having a selective affinity for the protein is fixed in the flow path of the microflow cell (microchip) in advance, the protein may be labelled with an enzyme using the principle of the Enzyme-Linked Immunosorbant Assay, and a quantitative measurement may be made using a coloring reaction with the enzyme. A microflow cell (microchip) for LDH detection and a microflow cell (micro chip) for antibody detection may be placed so that this antibody detection can be carried out in parallel with the detection of LDH described above.

The measurement unit according to the present invention includes means for feeding a plurality of reagents through a very small flow path, a displaceable very small flow path, a light source, an optical filter transmissive to only light of a specified wavelength of the light emitted from the light source, an optical cell for pouring a colored solution by an enzymatic reaction, and a light-receiving element for measuring the intensity of light transmitting through the reagent in the optical cell.

Furthermore, liquid feed switching means is provided between the microflow cell (microchip) and the optical cell, so that liquid feed to the optical cell or a waste liquid tank can be selected.

According to the present invention, since the sample liquid from each step is collected online, there is no possibility that the external atmosphere and the culture liquid in a culture tank come into contact and that bacteria enter each apparatus of a cell culture step, a separation step and a purification step, and thus destruction of biological cells or degradation of the sample liquid in each step can be prevented. Furthermore, if the sample liquid is automatically collected instead of manual collection of the sample liquid by an operator, the burden on the operator can be considerably reduced.

Furthermore, since the collected sample liquid is diluted to adjust liquid conditions such as the salt concentration and pH, stability of the antigen-antibody reaction in the measurement unit can be improved, measurement errors can be reduced, and filtration can be eased.

Furthermore, by filtering the sample liquid, impurities can be prevented from being fed to the measurement unit, thus making it possible to protect a microflow cell (microchip) from blocking of a micro-channel and so, improve measurement accuracy. Furthermore, because of the measurement method using the antigen-antibody reaction, it is not necessary to pass the sample liquid through a fine column as in high pressure liquid chromatography (HPLC) that is a conventional method, and the necessity to use a high pressure pump and pressure resistance specifications can be eliminated, thus making it possible to achieve downsizing of apparatus and a reduction in cost.

According to another embodiment of the present invention, the automatic protein measurement apparatus is characterized in that a protein is quantitatively measured by making the antigen-antibody reaction proceed in a very small flow path having a longitudinal cross-sectional area of 0.04 mm² or less in the measurement unit. According to the present invention, the antigen-antibody reaction that is diffusion-controlled is made to proceed in a very small flow path having a longitudinal cross-sectional area of 0.04 mm² or less, and therefore diffusion time is reduced, thus making it possible to considerably reduce the time for quantitative measurement.

It takes 3 hours for analysis with a reaction field having a depth of about 2 mm in the conventional measurement method, but by reducing the size of the reaction field to a 0.2 mm x 0.2 mm square or less that is equivalent to 1/10 of that of the conventional method, the time required for measurement can be reduced to 1/100, and hence to a desired time of 5 minutes or less, thus making it possible to perform monitoring of the culture state and high quality product control.

According to still another automatic protein measurement apparatus any one of fluorometry, absorptiometry and phosphorometry is used as means for detecting a protein. According to the present invention, if absorptiometry is used as detection means, the apparatus can be downsized. This is because optical measurements can be made with a configuration of small simple devices such as a light source, an optical filter and a light-receiving element.

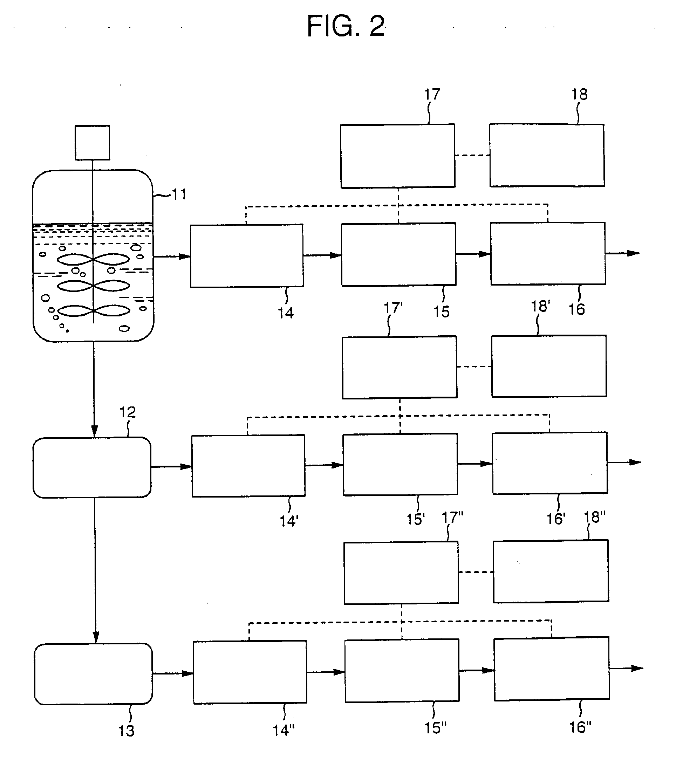

Embodiments of the present invention will be described in detail below with reference to the drawings. Fig. 2 is a system diagram of the automatic protein measurement apparatus. As shown in Fig. 2, the apparatus includes the following apparatus configuration.
(1) Online sampling units 14, 14' and 14" for automatically collecting a sample liquid from at least one operation step of a cell culture step (wherein an antibody or other proteins are produced and secreted into the culture medium) 11 and a separation process step 12 or a culture product purification step 13 in a plant for culturing biological cells to produce a protein, or tubes and storage before and after the respective steps and the like.
   Thus, a sample is collected from a flow including at least the above three steps. The sample may be further collected from other steps or before or after the steps as a matter of course.
(2) Preprocessing units 15, 15' and 15" for performing at least one of dilution and filtering of the sample liquid to adjust the liquid composition so that the sample liquid can be passed through the microflow cell (microchip).
(3) Measurement units 16, 16' and 16" for making a protein contained in the adjusted liquid emit light using the antigen-antibody reaction using the microflow cell (microchip) to quantitatively measure the protein. A light emitting signal can easily be detected using an optical system.
(4) Control units 17, 17' and 17" for automatically controlling a series of operations of the apparatus with the above configuration.
(5) Recording units 18, 18' and 18" for recording results of quantitative measurements.

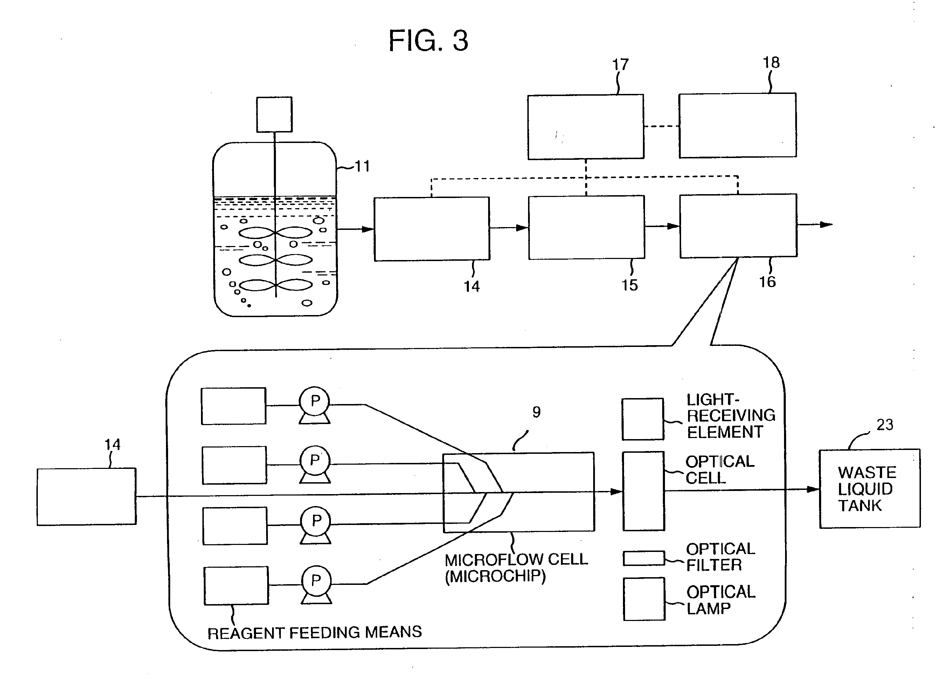

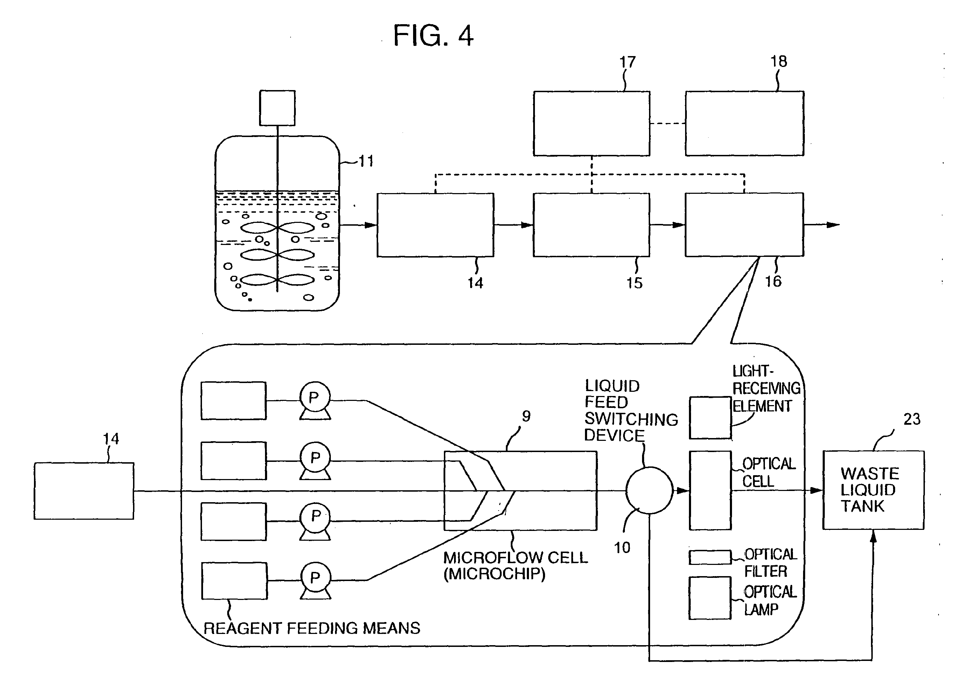

Figs. 3 and 4 illustrate in detail a measurement system including the microflow cell (microchip). In Figs. 3 and 4, like symbols denote like elements, and symbols same as those of Fig. 2 denote like elements.

In Figs. 3 and 4, in the measurement unit 16, the sample collected in the sampling unit 14 is fed to the microflow cell (microchip), and a reagent(s) required for the sample are fed by a pump P. A protein of interest in the sample is labelled with an enzyme after adsorbing to a primary antibody which is fixed on polymer beads filled in the micro-channel of the microflow cell (microchip) 9, and the enzyme reacts with a reagent(s) fed by a pump P to develop a color. The sample is coupled to an optical cell together with the beads, and detected. After the detection, beads and sample are fed to a waste liquid tank.

Fig. 4 shows an apparatus configuration where a liquid containing the protein, of the sample liquid fed to the microflow cell (microchip) 9, is directly fed to the waste liquid tank by a liquid feed switching device 10 such as a rotary valve after the microflow cell (microchip) 9. Since the liquid containing the protein can be fed without being passed through the optical cell, deposition of the protein on the optical cell can be prevented, thus making it possible to reduce errors in the measurement result.

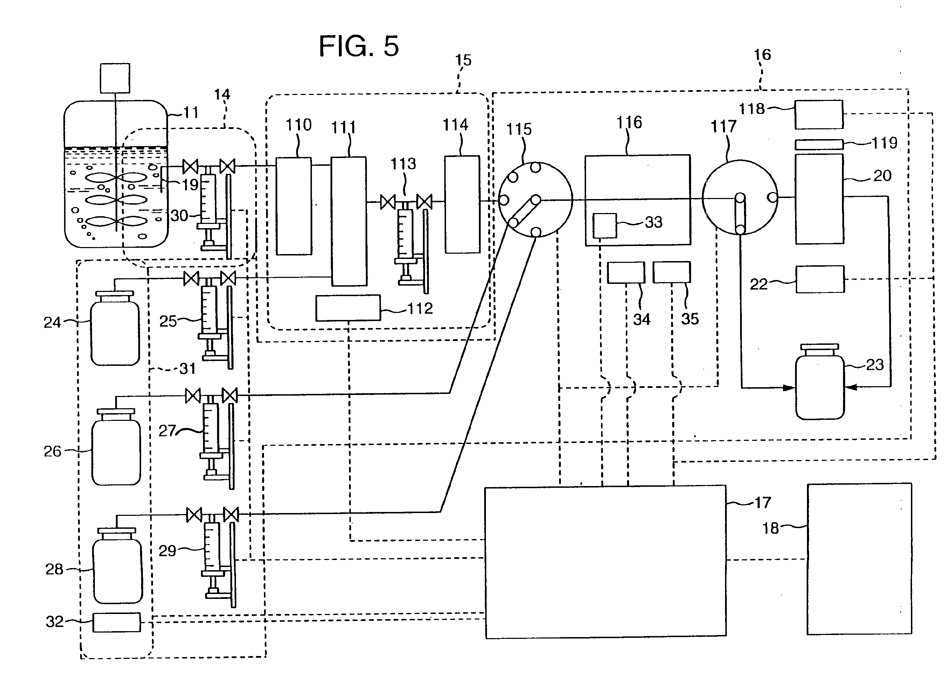

Fig. 5 shows one example of an apparatus configuration where a culture solution is collected from a culture tank, and a protein is quantitatively measured by the ELISA method. Operations beginning with the step of collecting the culture solution from the culture step and quantitatively measuring the protein by the ELISA method using the microflow cell (microchip), and ending with the step of recording the result will be described using Fig. 5.

Furthermore, proteins to be measured by the quantitative measurement apparatus include not only production proteins that are secreted into the culture solution as biological cells grow, but also enzyme-proteins such as LDH that are flowed out from cells into the culture solution after death of the biological cells.

First, the culture solution is collected online from a sampling tube 19 connected to a culture tank 11 using a pump 30 so that the external atmosphere and the culture solution do not come into contact, and the collected culture solution is fed to the preprocessing unit 15. Since automatic collection is carried out online unlike a conventional manual collection, no bacteria can enter from the external atmosphere. Thus, degradation of the sample liquid can be prevented, and the burden on the operator can be reduced. The pump is preferably a syringe pump in the sense that errors in the results of quantitative measurements are inhibited by reducing pulsation when the liquid is fed, but a piezo pump may be used.

The culture solution fed to the preprocessing unit 15 has impurities such as cell tissues, debris, etc., which are first roughly removed therefrom by a coarse filter 110, and is then fed to a dilution/mixing cell 111. At the same time, a specified amount of diluted liquid is fed from a diluted liquid tank 24 to the dilution/mixing cell 111 using a pump 25. The two liquids fed to the dilution/mixing cell 111 are mixed uniformly by a mixer 112, and then fed to precision filter 114 using a pump 27, and the sample liquid adjusted for measurement is fed to the measurement unit 16.

Since impurities such as cell tissues are filtered away in advance by the coarse filter 110, the reagent can easily be diluted in the dilution/mixing cell 111. Furthermore, by using a pH buffer solution for a diluting liquid, the pH of the sample liquid can be stabilized, thus making it possible to stabilize the subsequent antigen-antibody reaction in the measurement unit 16 to reduce measurement errors.

For the mixer 112, an ultrasonic vibration apparatus using a piezo element or a vibration mixer using a motor is desirably used in the sense that liquids are uniformly mixed in short time not in contact with the sample liquid, but a mixer using a magnetic stirrer may be used. Furthermore, since impurities that may cause clogging of the very small flow path of the measurement unit 16 in the subsequent stage are removed by the precision filter 114, the reagent flow in the very small flow path can be stabilized, thus making it possible to reduce measurement errors due to stabilization of the reaction. Between the precision filter 114 and the reaction tank 116 a liquid feed switching device 115 is provided.

In addition to the sample liquid adjusted in the preprocessing unit 15, reagents required for measurement by the ELISA method are fed to the measurement unit 16 from reagent tanks 26, 28 using pumps 27, 29. First, the adjusted sample liquid is made to flow through the microflow cell (microchip) (reaction tank) 116 in which a primary antibody for a protein (antigen) to be measured is immobilized in a solid phase in advance in a very small flow path having a sectional-area of 1 mm² or less, and coupled to the primary antibody. A secondary antibody liquid and an enzyme liquid are sequentially fed and coupled, and then a coloring reagent solution is made to flow.

The reagent solution contacts the coloring enzyme to develop a color in the measurement unit 16, and is fed to an optical cell 20 in the subsequent stage.

Light of a wavelength that is absorbed by a coloring reagent, of light produced in an optical lamp 118, is selected with an optical filter 119, and passed through the optical cell 20. The coloring absorbance of transmitting light is measured with a light-receiving element 22, and then the reagent solution is fed to a waste liquid tank 23.

Furthermore, since reagents other than the matrix solution contain proteins, measurement errors may occur due to adsorption of proteins on the cell surface if these reagents are fed to the optical cell 20. Therefore, reagents other than the matrix solution are selected and fed directly to the waste liquid tank by a liquid feed switching device 117.

For the liquid feed switching devices 115 and 117, a rotary valve having a low dead volume may be used alone, or a branched very fine flow path may be provided in the reaction tank 116 to provide a liquid feed selection function in the reaction tank 116. If the rotary valve having a low dead volume is used, contamination among reagents can be prevented, thus making it possible to improve measurement accuracy. Furthermore, if the branched very small flow path is used, it is possible to prevent degradation of the rotary valve caused by adsorption of the protein on the liquid contact surface.

One example of the reaction tank 116 is a micro reactor described in JP-A-2001-4628. It takes 3 hours for analysis with a reaction field having a depth of about 2 mm in the conventional measurement method, but by reducing the size of reaction field to a 0.02 mm square or less that is equivalent to 1/10 of that of the conventional method, the time required for measurement is reduced to 1/100, which means that it can be reduced to 5 minutes or less, thus making it possible to perform monitoring of the culture state and high quality product control.

The post-measurement sample liquid is fed to the waste liquid tank (pool) 23 to complete the analysis. Furthermore, the temperature of the reaction tank is measured by a temperature sensor 33, and temperature control is performed by a heater or Peltier element 34. Furthermore, because liquids in the diluted liquid tank 24 and reagent tanks 26, 28 are easily degraded depending upon temperatures, temperature control is performed with the temperature sensor 33 and a cooler 31 so that those liquids can be stored at a temperature of 2 to 10°C.

Feed of the sample liquid and reagents by the pump, activation of the mixer of the dilution cell, operation of the liquid feed switching device, adjustment of temperature of the reaction tank, adjustment of temperature of a reagent repository, output of the measurement results and control of the optical system are all performed by a controller. The result of optical measurement is output to a recording unit 18.

According to the example described above, the sample liquid is collected online, and therefore there is no possibility that the external atmosphere and the culture liquid in a culture tank can come into contact and that bacteria enter any apparatus of a cell culture step, a separation step and a purification step. Therefore, destruction of biological cells in the culture process, or degradation of the sample liquid in the separation/refinement process can be prevented. Furthermore, the sample liquid is automatically collected, the burden on an operator can be considerably reduced.

Furthermore, since the liquid conditions, such as the salt concentration and pH, of the collected sample liquid are adjusted in the preprocessing unit, stability of the antigen-antibody reaction can be improved, and measurement errors can be reduced. Furthermore, because of the measurement method using the antigen-antibody reaction, it is not necessary to pass the sample liquid through a fine column as in the conventional high pressure liquid chromatography (HPLC).

As a result, the necessity to use a high pressure pump and pressure resistance piping specifications can be eliminated, thus making it possible to achieve downsizing of the apparatus and a reduction in cost.

Furthermore, since the antigen-antibody reaction that is diffusion-controlled is made to proceed in a very small flow path having a longitudinal cross-sectional area of 1 mm² or less, the diffusion time is reduced to 1/9 of that of the conventional method, and thus the time for quantitative measurement can be reduced to 20 minutes or less. Furthermore, this makes it possible to perform monitoring of culture states and high quality product control.

Furthermore, since any of fluorometry, absorptiometry and phosphorometry is used as means for detecting a protein, optical measurements can be made with a combination of small simple devices such as a light source, an optical filter and a light-receiving element. Thus, downsizing of the apparatus is possible.

According to the example described above, the sample liquid is collected online, thus making it possible to prevent contact between the external atmosphere and the culture liquid. Consequently, entrance of bacteria into the apparatus can be prevented, thus making it possible to prevent destruction of biological cells in the culture process, or degradation of the sample liquid in the separation/refinement process.

Because of automatic collection, the burden on an operator can be considerably reduced. Since the liquid conditions, such as the salt concentration and pH, of the collected sample liquid are adjusted in the preprocessing unit, the stability of the antigen-antibody reaction can be improved, and measurement errors can be reduced.

Because of the measurement method using the antigen-antibody reaction, it is not necessary to pass the sample liquid through a fine column as in the conventional HPLC method, and therefore the necessity to use a high pressure pump and pressure resistance piping specifications can be eliminated, thus making it possible to achieve downsizing of apparatus and a reduction in cost.

Furthermore, since the antigen-antibody reaction that is diffusion-controlled is made to proceed in a very small flow path having a longitudinal cross-sectional area of 0.04 mm² or smaller, diffusion time is reduced to 1/100 of that of the conventional method, and thus the time for quantitative measurement can be reduced to 5 minutes or less. Furthermore, this makes it possible to perform monitoring of culture states and high quality product control.

Furthermore, since any of fluorometry, absorptiometry and phosphorometry is used as means for detecting a protein, optical measurements can be made with a combination of small simple devices such as a light source, an optical filter and a light-receiving element, thus making it possible to achieve downsizing of the apparatus.

Since the sample liquid is collected online, there is no possibility that the external atmosphere and the sample liquid can come into contact and that bacteria enter any apparatus of a cell culture step, a separation step and a purification step, thus making it possible to prevent destruction of biological cells in the culture process, or degradation of the sample liquid in the separation/refinement process.

Table 1 shows the time required for analysis when mouse-mouse hybridoma STK-1 cells capable of secreting and producing a DNA polymerase α antibody (IgG) were cultured in a 11 culture tank, and the concentration of antibody in the culture solution was measured by the method of the present invention and the conventional ELISA method.

In the measurement method of the present invention, chicken anti-mouse IgG was immobilized in a micro-flow path in advance, and a sample liquid containing IgG was made to flow therethrough at a rate of 50 µl/min, followed by cleaning with a bovine serum albumin solution. Then, biotin-labelled horse anti-mouse IgG was made to flow therethrough, the bovine serum albumin solution was made to flow again, and then avidin-labelled alkali phosphatase was made to flow.

The bovine serum albumin solution was made to flow for cleaning; then p-nitrophenyl phosphate was made to flow, and the coloring absorbance was measured at 405 nm. Furthermore, in the conventional ELISA method, similarly a culture solution containing mouse IgG was injected into a well plate having chicken anti-mouse IgG adsorbed on the bottom in advance; the plate was cleaned; then the plate was cleaned with a bovine serum albumin solution, and biotin-labelled horse anti-mouse IgG was made to flow therethrough. Then, the bovine serum albumin solution was made to flow; then avidin-labelled alkali phosphatase was made to flow; the bovine serum albumin solution was made to flow therethrough for cleaning; then p-nitrophenyl phosphate was made to flow, and the coloring absorbance was measured at 405 nm.

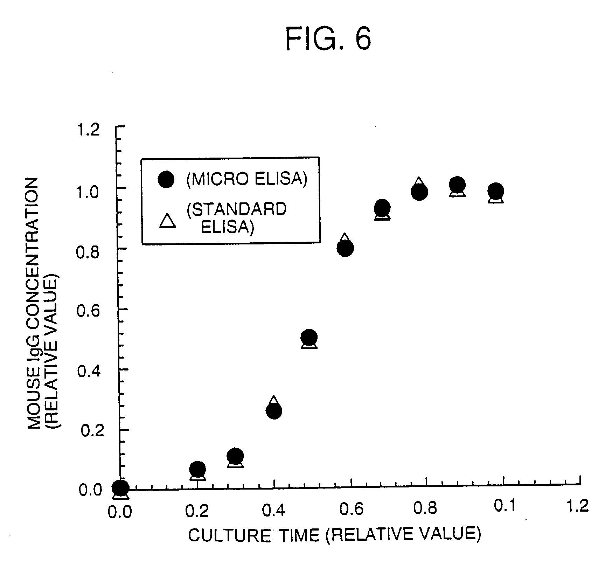

As a result, the conventional ELISA method required several hours for analysis, while in the analysis method of the present invention, a measurement could be made in about 5 minutes, and the amount of sample liquid could be reduced to 1/10 or less. The comparison of the measurement results of both methods in Fig. 6 shows that the measurement performance obtained by the measurement method of the present invention is equivalent to that obtained by the conventional ELISA method.

**[Table 1]**

| Analysis flow | Micro ELISA method | | Standard ELISA method | |
|---|---|---|---|---|
| | Required time | Liquid amount* | Required time | Liquid amount |
| 1) Target protein (IgG) coupling | 1 min | 50 µl | 60 min | 50 µl |
| Plate cleaning | 1 min | 50 µl | 5 min | 1200 µl |
| 2) Secondary antibody coupling | 1 min | 50 µl | 30 min | 50 µl |
| Plate cleaning | 1 min | 50 µl | 5 min | 1200 µl |
| 3) Enzyme labeling | 1 min | 50 µl | 30 min | 50 µl |
| Plate cleaning | 1 min | 50 µl | 5 min | 1200 µl |
| 4) Coloring enzyme reaction start Measurement of coloring absorbance (measurement wavelength 405 nm) | 0.5 min | 25 µl | 3 min. | 100 µl |
| Total | 6.5 min | 325 µl | 138 min | 3850 µl |

| | | | | |
|---|---|---|---|---|
| *Liquid flow rate: 50 µl/min | | | | |

According to the present invention, a quantitative measurement of a protein can be made in short time with a simple apparatus configuration, and contamination of a sample can be prevented, thus making it possible to perform highly reliable analysis.

### List of the reference numerals

- 1: upper substrate
- 2: lower substrate
- 3: microchannel
- 4: microchannel
- 5: tube
- 6: tube
- 7: tube
- 8: tube
- 9: microflow cell (microchip)
- 10: liquid feed switching device
- 11: culture tank
- 12: separation process step
- 13: culture product purification step
- 14, 14', 14": online sampling units
- 15, 15', 15": preprocessing units
- 16, 16', 16": measurement units
- 17, 17', 17": control units
- 18, 18', 18": recording units
- 19: sampling tube
- 20: optical cell
- 22: light receiving element
- 23: waste liquid tank
- 24: diluted liquid tank
- 25: pump
- 26: reagent tank
- 27: reagent pump
- 28: reagent tank
- 29: reagent pump
- 30: pump
- 31: cooler
- 33: temperature sensor
- 34: heater (Peltier element)
- 35: cooler
- 110: coarse filter
- 111: dilution/mixing cell
- 112: mixer
- 113: pump
- 114: precision filter
- 115: liquid feed switching device
- 116: microflow cell (microchip) (reaction tank)
- 117: liquid feed switching device
- 118: optical lamp
- 119: optical filter

## Claims

1. Method for the quantitative measurement of proteins in a liquid sample,
comprising the following steps:
- sampling a liquid sample, preferably online,
- diluting the sample to adjust pH, ionic strength, osmolality, etc., of the sample,
- passing the liquid sample through a microflow cell (microchip) having a micro-channel constituting a flow path of the microflow cell,
- reacting the protein contained in the liquid sample to achieve a coloring reaction with an enzyme or a reaction yielding a product showing fluorescence or phosphorescence,
and
- measuring the amount of protein,
**characterized in that** the cross-sectional area of the micro-channel is 0.04 mm² or less and **in that** the step of passing the liquid sample is preceded by a step of immobilizing a primary antibody having a selective affinity for the protein in the flow path of the microflow cell (microchip) in advance.

2. Method for the quantitative measurement of a protein in a liquid sample according to claim 1, further comprising:
. a cell culture step, wherein proteins, such as antibodies, enzymes or other proteins, are produced and secreted into the culture solution or are flowed out from dead or disrupted cells into the culture solution,
. a separation step subsequent to the cell culture step and
. a culture product purification step.

3. Method according to claim 1 or 2, wherein at least the steps of sampling the liquid sample, performing a dilution and/or filtration and passing the sample liquid through the microflow cell are performed in a closed system without contact to the outer atmosphere or other sources of impurities or biological contamination.

4. Method according to claim 3, wherein all of the steps including the measuring steps are performed in a closed system.

5. Method according to any of claims 1 to 4, wherein a disposable microflow cell (microchip) is used.

6. Method according to any of claims 1 to 5, wherein a microflow cell (microchip) is used having a cross-sectional area of a reaction area of 0.04 mm² or less.

7. Apparatus for the quantitative measurement of proteins in liquid samples,
preferably for carrying out the methods of claims 1 to 6,
comprising:
- a sampling unit (14, 14', 14") for taking a sample liquid online at least one time from a flow including
. a cell culture step, wherein proteins, such as antibodies, enzymes or other proteins, are produced and secreted into the culture medium or are flowed out from dead or disrupted cells into the culture medium,
. a separation step subsequent to the cell culture step,
and
. a culture product purification step,
- a preprocessing unit (15, 15', 15") for performing a dilution and/or filtration of the sample liquid to adjust the liquid composition so that the sample liquid can be passed through a microflow cell (9; 116),
- a microflow cell (microchip) (9; 116) having a micro-channel constituting a flow path of the microflow cell for making a protein contained in the sample liquid obtained from the preprocessing unit (15, 15', 15") undergo a coloring reaction with an enzyme or a reaction resulting in a product showing fluorescence or phosphorescence,
- a measurement unit (16, 16', 16") for quantitatively measuring the protein using a reaction result,
and
- a control unit (17, 17', 17") for controlling the operation of at least one of the sampling unit (14, 14', 14"), the preprocessing unit (15, 15', 15"), the microflow cell (9; 116) and the measurement unit (16, 16', 16")
**characterized in that** the cross-sectional area of the micro-channel is 0.04 mm² or less and **in that** a primary antibody having a selective affinity for the protein is immobilized in the flow path of the microflow cell (microchip) in advance.

8. Apparatus according to claim 7, further comprising a recording unit (18, 18', 18") for recording quantitative measurement results.

9. Apparatus according to claim 7 or 8, wherein the control unit (17, 17', 17") is designed to control all of the operations of the apparatus.

10. Apparatus according to any of claims 7 to 9, wherein the microflow cell (microchip) (9; 116) is disposable.

11. Apparatus according to any of claims 7 to 10, wherein the microflow cell (microchip) (9; 116) has a cross-sectional area of a reaction area of 0.04 mm² or less.

12. Apparatus according to any of claims 7 to 11, further comprising:
- at least one reagent tank (26, 28),
- at least one diluted liquid tank (24),
- at least one pump (30) for pumping culture medium from a culture tank (11) into the preprocessing unit (15),
- at least one pump (25) for pumping diluted liquid from a diluted liquid tank (24) to the preprocessing unit (15), and
- at least one reagent pump (27, 29) for pumping reagent into the measurement unit (16).

13. Apparatus according to any of claims 7 to 12, wherein the preprocessing unit (15) comprises:
- a coarse filter (110),
- a subsequent dilution/mixing cell (111),
- a pump (113) for pumping sample liquid from the dilution/mixing cell (111) to a subsequent precision filter (114).

14. Apparatus according to any of claims 7 to 13, wherein the measurement unit (16) comprises:
- a liquid feed switching device (115) connected to the outlet of the precision filter (114) and to reagent pumps (27, 29) for switching the flow through the microflow cell (microchip) (116),
- a microflow cell (microchip) (116) provided with a temperature sensor (33) and a heater and/or cooler (34, 35),
- a liquid feed switching device (117) for switching the flow path from the microflow cell (116) to an optical cell (20) to a waste liquid tank (23).

15. Apparatus according to any of claims 7 to 14, further including an optical measurement unit (118, 119, 20, 22) comprising:
- a light source (118),
- an optical filter (119),
- an optical cell (20),
and
- a light receiving element (22).

16. Apparatus according to any of claims 7 to 15, wherein the components of the flow path form a closed system without contact to the outer atmosphere or other sources of impurities or biological contamination.

## Patentansprüche

1. Verfahren zur quantitativen Messung von Proteinen in einer flüssigen Probe, welches folgende Schritte umfasst:
das Entnehmen einer flüssigen Probe, vorzugsweise online,
das Verdünnen der Probe zum Zwecke der Anpassung von pH-Wert, Ionenstärke, Osmolarität, usw. der Probe,
das Leiten der flüssigen Probe durch eine Mikrodurchflusszelle (Mikrochip), welche über einen Mikrokanals verfügt, der den Fließweg der Mikrodurchflusszelle darstellt,
das Reagieren des in der flüssigen Probe enthaltenen Proteins,
um eine Farbreaktion mit einem Enzym hervorzurufen oder eine Reaktion, welche ein Produkt erzeugt, das Fluoreszenz oder Phosphoreszenz zeigt,
sowie
das Messen der Proteinmenge,
**dadurch gekennzeichnet, dass** die Querschnittsfläche des Mikrokanals 0,04 mm² oder weniger beträgt und dass dem Schritt des Durchleitens der flüssigen Probe ein Schritt vorausgeht, in dem ein Primärantikörper, welcher eine selektive Affinität für das Protein aufweist, in dem Fließweg der Mikrodurchflusszelle (Mikrochip) im Voraus immobilisiert wird.

2. Das Verfahren zur quantitativen Messung von Proteinen in einer flüssigen Probe nach Anspruch 1, welches weiter folgendes umfasst:
einen Zellkulturen-Schritt, in dem Proteine, wie etwa Antikörper, Enzyme oder andere Proteine, hergestellt und in die Kulturlösung sekretiert werden, oder aus toten oder aufgeschlossenen Zellen in die Kulturlösung fließen,
ein Trennungsschritt, welcher auf den Zellkulturschritt folgt, sowie
ein Kulturprodukt-Reinigungsschritt.

3. Das Verfahren nach Anspruch 1 oder 2, wobei mindestens die Schritte der Entnahme einer flüssigen Probe, des Durchführens eines Verdünnens und/oder Filterns und des Durchleitens der flüssigen Probe durch die Mikroflusszelle in einem geschlossenen System durchgeführt werden ohne Kontakt zur Außenatmosphäre oder anderen Quellen von Verunreinigungen oder biologischer Kontamination.

4. Das Verfahren nach Anspruch 3, wobei alle Schritte einschließlich der Messschritte in einem geschlossenen System durchgeführt werden.

5. Das Verfahren nach irgendeinem der Ansprüche 1 bis 4, wobei eine Einmal-Mikrodurchflusszelle (Mikrochip) verwendet wird.

6. Das Verfahren nach irgendeinem der Ansprüche 1 bis 5, wobei eine Mikrodurchflusszelle (Mikrochip) verwendet wird, welche einen Reaktionsbereich aufweist, dessen Querschnittsfläche 0,04 mm² oder weniger beträgt

7. Eine Vorrichtung zur quantitativen Messung von Proteinen in flüssigen Proben, vorzugsweise für die Durchführung der Methoden aus den Ansprüchen 1 bis 6, welche folgendes umfasst:
eine Probenahmeeinheit (14, 14', 14") für das mindestens einmalige Entnehmen von Probeflüssigkeit online aus einem Fluss, folgendes einschließend:
einen Zellkulturen-Schritt, in dem Proteine, wie etwa Antikörper, Enzyme oder andere Proteine, hergestellt und in die Kulturlösung sekretiert werden, oder aus toten oder aufgeschlossenen Zellen in die Kulturlösung fließen,
einen Trennungsschritt, welcher auf den Zellkulturschritt folgt,
sowie
einen Kulturprodukt-Reinigungsschritt,
eine Vorverarbeitungseinheit (15, 15', 15") für das Durchführen eines Verdünnens und/oder Filterns der flüssigen Probe zum Zwecke der Anpassung der flüssigen Zusammensetzung, sodass die flüssige Probe durch eine Mikrodurchflusszelle (9; 116) geleitet werden kann,
eine Mikrodurchflusszelle (Mikrochip) (9; 116), welche einen Mikrokanal aufweist, der den Fließweg der Mikrodurchflusszelle darstellt, für das Hervorrufen einer Farbreaktion eines in einer flüssigen Probe enthalten Proteins aus der Vorverarbeitungseinheit (15, 15', 15') mit einem Enzym oder einer Reaktion, welche dazu führt, dass das Produkt Fluoreszenz oder Phosphoreszenz zeigt,
eine Messeinheit (16, 16', 16") für die quantitativen Messung von Protein mit Hilfe eines Reaktionsergebnisses,
und
eine Steuerungseinheit (17, 17', 17") für das Steuern des Betriebs von mindestens einem der folgenden: der Probenahmeeinheit (14, 14', 14"), der Vorverarbeitungseinheit (15, 15', 15"), der Mikrodurchflusszelle (9; 116) und der Messeinheit (16, 16', 16"),
**dadurch gekennzeichnet, dass** die Querschnittsfläche des Mikrokanals 0,04 mm² oder weniger beträgt, und dass ein Primärantikörper, welcher eine selektive Affinität für das Protein aufweist, in dem Fließweg der Mikrodurchflusszelle (Mikrochip) im Voraus immobilisiert wird.

8. Die Vorrichtung nach Anspruch 7, welche weiter eine Aufzeichnungseinheit (18, 18', 18") für das Aufzeichnen der quantitativen Messergebnisse enthält.

9. Die Vorrichtung nach Anspruch 7 oder 8, wobei die Steuerungseinheit (17, 17', 17") derart gestaltet ist, dass sie alle Betriebsvorgänge der Vorrichtung steuert.

10. Die Vorrichtung nach irgendeinem der Ansprüche 7 bis 9, wobei die Mikrodurchflusszelle (Mikrochip) (9; 116) eine Einmal-Mikrodurchflusszelle darstellt.

11. Die Vorrichtung nach irgendeinem der Ansprüche 7 bis 10, wobei die Mikrodurchflusszelle (Mikrochip) (9; 116) einen Reaktionsbereich aufweist, dessen Querschnittsfläche 0,04 mm² oder weniger beträgt.

12. Die Vorrichtung nach irgendeinem der Ansprüche 7 bis 11, welche weiter folgendes umfasst:
mindestens einen Reagenzbehälter (26, 28),
mindestens einen Behälter für verdünnte Flüssigkeiten (24),
mindestens eine Pumpe (30) für das Pumpen des Kulturmediums von dem Kulturenbehälter (11) in die Vorverarbeitungseinheit (15),
mindestens eine Pumpe (25) für das Pumpen von verdünnten Flüssigkeiten von dem Behälter für verdünnte Flüssigkeiten (24) zu der Vorverarbeitungseinheit (15), und
mindestens eine Reagenzpumpe (27, 29), für das Pumpen der Reagenz in die Messeinheit (16).

13. Die Vorrichtung nach irgendeinem der Ansprüche 7 bis 12, wobei die Vorverarbeitungseinheit (15) folgendes umfasst:
einen Grobfilter (110),
eine nachgelagerte Verdünnungs-/Mischzelle (111),
eine Pumpe (113) für das Pumpen von Probenflüssigkeit von der Verdünnungs-/Mischzelle (111) zu einem nachgelagerten Präzisionsfilter (114).

14. Die Vorrichtung nach irgendeinem der Ansprüche 7 bis 13, wobei die Messeinheit (16) folgendes umfasst:
eine Flüssigkeitszufuhr-Schalteinrichtung (115), welche mit dem Auslass des Präzisionsfilters (114) sowie mit den Reagenzpumpen (27, 29) verbunden ist und dem Umschalten des Durchflusses durch die Mikrodurchflusszelle (Mikrochip) (116) dient,
eine Mikrodurchflusszelle (Mikrochip) (116), welche mit einem Temperatursensor (33) sowie mit einer Heizung und/oder Kühlung (34, 35) ausgestattet ist,
eine Flüssigkeitszufuhr-Schalteinrichtung (117) für das Umschalten des Fließwegs von der Mikrodurchflusszelle (116) zu einer optischen Zelle (20) zu einem Entsorgungstank (23).

15. Die Vorrichtung nach irgendeinem der Ansprüche 7 bis 14, welche weiter eine optische Messeinheit (118, 119, 20, 22) beinhaltet, welche folgendes umfasst:
eine Lichtquelle (118),
einen optischen Filter (119),
eine optische Zelle (20),
und
ein Lichtempfangselement (22).

16. Die Vorrichtung nach irgendeinem der Ansprüche 7 bis 15, wobei die Komponenten des Fließwegs ein geschlossenes System bilden ohne Kontakt zur Außenatmosphäre oder anderen Quellen von Verunreinigungen oder biologischer Kontamination.

## Revendications

1. Procédé de mesure quantitative de protéines dans un échantillon de liquide,
comprenant les étapes suivantes :
- échantillonnage d'un échantillon de liquide, préférablement en ligne,
- dilution de l'échantillon pour ajuster le pH, la force ionique, l'osmolalité, etc., de l'échantillon,
- passage de l'échantillon de liquide à travers une cellule à micro-débit (laboratoire sur puce) ayant un micro-canal constituant un chemin d'écoulement de la cellule à micro-débit,
- réaction de la protéine contenue dans l'échantillon de liquide pour obtenir une réaction de coloration avec une enzyme ou une réaction donnant un produit montrant une fluorescence ou une phosphorescence,
et
- mesure de la quantité de protéine,
**caractérisé en ce que** la surface de section transversale du micro-canal est 0,04 mm² ou moins et **en ce que** l'étape de passage de l'échantillon de liquide est précédée d'une étape d'immobilisation d'un anticorps primaire ayant une affinité sélective pour la protéine dans le chemin d'écoulement de la cellule à micro-débit (laboratoire sur puce) au préalable.

2. Procédé de mesure quantitative d'une protéine dans un échantillon de liquide selon la revendication 1, comprenant en outre :
• une étape de culture de cellules, dans laquelle des protéines, telles que des anticorps, des enzymes ou autres protéines, sont produites et sécrétées dans la solution de culture ou sont évacuées à partir de cellules mortes ou détruites dans la solution de culture,
• une étape de séparation faisant suite à l'étape de culture de cellules et
• une étape de purification de produit de culture.

3. Procédé selon la revendication 1 ou 2, dans lequel au moins les étapes d'échantillonnage de l'échantillon de liquide, d'exécution d'une dilution et/ou d'une filtration et de passage du liquide échantillon à travers la cellule à micro-débit sont mises en oeuvre dans un système fermé sans contact avec l'atmosphère extérieure ou autres sources d'impuretés ou de contamination biologique.

4. Procédé selon la revendication 3, dans lequel toutes les étapes, incluant les étapes de mesure, sont mises en oeuvre dans un système fermé.

5. Procédé selon l'une quelconque des revendications 1 à 4, dans lequel une cellule à micro-débit (laboratoire sur puce) jetable est utilisée.

6. Procédé selon l'une quelconque des revendications 1 à 5, dans lequel une cellule à micro-débit (laboratoire sur puce) est utilisée, ayant une surface de section transversale d'une surface de réaction de 0,04 mm² ou moins.

7. Appareil pour la mesure quantitative de protéines dans des échantillons de liquide,
préférablement pour mettre en oeuvre les procédés des revendications 1 à 6,
comprenant :
- une unité (14, 14', 14") d'échantillonnage pour prendre un liquide échantillon en ligne au moins une fois à partir d'un écoulement incluant
• une étape de culture de cellules, dans laquelle des protéines, telles que des anticorps, des enzymes ou autres protéines, sont produites et sécrétées dans la solution de culture ou sont évacuées à partir de cellules mortes ou détruites dans la solution de culture,
• une étape de séparation faisant suite à l'étape de culture de cellules,
et
• une étape de purification de produit de culture,
- une unité (15, 15', 15") de prétraitement pour exécuter une dilution et/ou une filtration du liquide échantillon pour ajuster la composition de liquide de façon à ce que le liquide échantillon puisse être passé à travers une cellule à micro-débit (9 ; 116),
- une cellule à micro-débit (laboratoire sur puce) (9 ; 116) ayant un micro-canal constituant un chemin d'écoulement de la cellule à micro-débit pour faire en sorte qu'une protéine contenue dans le liquide échantillon obtenu dans l'unité (15, 15', 15") de prétraitement subisse une réaction de coloration avec une enzyme ou une réaction résultant en un produit montrant une fluorescence ou une phosphorescence,
- une unité (16, 16', 16") de mesure pour mesurer quantitativement la protéine en utilisant un résultat de réaction,
et
- une unité (17, 17', 17") de commande pour commander le fonctionnement d'au moins une parmi l'unité (14, 14', 14") d'échantillonnage, l'unité (15, 15', 15") de prétraitement, la cellule à micro-débit (9 ; 116) et l'unité (16, 16', 16") de mesure
**caractérisé en ce que** la surface de section transversale du micro-canal est 0,04 mm² ou moins et **en ce qu'**un anticorps primaire ayant une affinité sélective pour la protéine est immobilisé dans le chemin d'écoulement de la cellule à micro-débit (laboratoire sur puce) au préalable.

8. Appareil selon la revendication 7, comprenant en outre une unité (18, 18', 18") d'enregistrement pour enregistrer des résultats de mesure quantitative.

9. Appareil selon la revendication 7 ou 8, dans lequel l'unité (17, 17', 17") de commande est conçue pour commander toutes les opérations de l'appareil.

10. Appareil selon l'une quelconque des revendications 7 à 9, dans lequel la cellule à micro-débit (laboratoire sur puce) (9 ; 116) est jetable.

11. Appareil selon l'une quelconque des revendications 7 à 10, dans lequel la cellule à micro-débit (laboratoire sur puce) (9 ; 116) a une surface de section transversale d'une surface de réaction de 0,04 mm² ou moins.

12. Appareil selon l'une quelconque des revendications 7 à 11, comprenant en outre :
- au moins un réservoir (26, 28) de réactif,
- au moins un réservoir (24) de liquide dilué,
- au moins une pompe (30) pour pomper un milieu de culture d'un réservoir (11) de culture jusque dans l'unité (15) de prétraitement,
- au moins une pompe (25) pour pomper un liquide dilué d'un réservoir (24) de liquide dilué jusque dans l'unité (15) de prétraitement, et
- au moins une pompe (27, 29) de réactif pour pomper un réactif dans l'unité (16) de mesure.

13. Appareil selon l'une quelconque des revendications 7 à 12, dans lequel l'unité (15) de prétraitement comprend :
- un filtre (110) grossier,
- une cellule (111) de dilution/mélange subséquente,
- une pompe (113) pour pomper un liquide échantillon de la cellule (111) de dilution/mélange jusqu'à un filtre (114) de précision subséquent.

14. Appareil selon l'une quelconque des revendications 7 à 13, dans lequel l'unité (16) de mesure comprend :
- un dispositif (115) de commutation d'alimentation de liquide connecté à la sortie du filtre (114) de précision et à des pompes (27, 29) de réactif pour commuter l'écoulement à travers la cellule à micro-débit (laboratoire sur puce) (116),
- une cellule à micro-débit (laboratoire sur puce) (116) prévue avec un capteur (33) de température et un dispositif de chauffage et/ou de refroidissement (34, 35),
- un dispositif (117) de commutation d'alimentation de liquide pour commuter l'écoulement de la cellule à micro-débit (116) jusqu'à une cellule (20) optique jusqu'à un réservoir (23) de déchet liquide.

15. Appareil selon l'une quelconque des revendications 7 à 14, incluant en outre une unité (118, 119, 20, 22) de mesure optique comprenant :
- une source (118) de lumière,
- un filtre (119) optique,
- une cellule (20) optique,
et
- un élément (22) de réception de lumière.

16. Appareil selon l'une quelconque des revendications 7 à 15, dans lequel les composants du chemin d'écoulement forment un système fermé sans contact avec l'atmosphère extérieure ou autres sources d'impuretés ou de contamination biologique.
